# EUROPEAN PATENT SPECIFICATION

(11) **EP 2 836 502 B1**
(45) Date of publication and mention of the grant of the patent: **02.08.2017**
(21) Application number: 13775974.2
(22) Date of filing: 15.03.2013
(51) Int. Cl.: C07J 31/00, A61K 31/575, A61P 3/06, A61P 29/00, A61P 3/10, A61P 9/10, A61P 1/16, C07J 9/00

(54) **A NOVEL CHOLESTEROL METABOLITE, 5-CHOLESTEN, 3beta-25-DIOL, DISULFATE (25HCDS) FOR THERAPY OF METABOLIC DISORDERS, HYPERLIPIDEMIA, DIABETES, FATTY LIVER DISEASES AND ATHEROSCLEROSIS**
NEUARTIGES CHOLESTERINMETABOLIT 5-CHOLESTEN, 3BETA-25-DIOL, DISULFAT (25HCDS) ZUR BEHANDLUNG VON STOFFWECHSELERKRANKUNGEN, HYPERLIPIDÄMIE, DIABETES, FETTLEBER UND ATHEROSKLEROSE
NOUVEAU MÉTABOLITE DU CHOLESTÉROL, 5-CHOLESTÈNE, 3beta-25-DIOL, DISULFATE (25HCDS) POUR LA THÉRAPIE DE TROUBLES MÉTABOLIQUES, DE L'HYPERLIPIDÉMIE, DU DIABÈTE, DES STÉATOSES HÉPATIQUES ET DE L'ATHÉROSCLÉROSE

(30) Priority: 12.04.2012 US 201261623414 P; 12.04.2012 US 201261623203 P
(43) Date of publication of application: 18.02.2015
(60) Divisional application: 17167122.5
(73) Proprietor: Virginia Commonwealth University, Richmond, VA 23219 (US)
(72) Inventor: REN, Shunlin, Redmond, VA 23249 (US)
(74) Representative: J A Kemp
(86) International application number: PCT/US2013/031861
(87) International publication number: WO 2013/154752

(56) References cited:
- WO-A1-2006/047022
- WO-A2-2011/077245
- US-A1- 2007 275 939
- I. T. COOK ET AL: "24-Hydroxycholesterol Sulfation by Human Cytosolic Sulfotransferases: Formation of Monosulfates and Disulfates, Molecular Modeling, Sulfatase Sensitivity, and Inhibition of Liver X Receptor Activation", DRUG METABOLISM AND DISPOSITION, vol. 37, no. 10, 9 July 2009 (2009-07-09), pages 2069-2078, XP55200547, DOI: 10.1124/dmd.108.025759
- REN, S. ET AL.: 'Sulfated oxysterol, 25HC3S, is a potent regulator of lipid metabolism in human hepatocytes' BIOCHEM. BIOPHYS. RES. COMMUN. vol. 360, no. 4, 06 July 2007, pages 802 - 808, XP025990001
- XU, L. ET AL.: '25-Hydroxycholesterol-3-sulfate attenuates inflammatory response via PPAR signaling in human THP-1 macrophages' AM. J. PHYSIOL. ENDOCRINOL. METAB. vol. 302, no. 7, 24 January 2012, pages E788 - E799, XP055162856
- MA, Y. ET AL.: '25-Hydroxycholesterol-3-sulfate regulates macrophage lipid metabolism via the LXR/SREBP-1 signaling pathway' AM. J. PHYSIOL. ENDOCRINOL. METAB. vol. 295, no. 6, 14 October 2008, pages E1369 - E1379, XP055162858
- XU, L. ET AL.: 'Regulation of hepatocyte lipid metabolism and inflammatory response by 25-hydroxycholesterol and 25-hydroxycholesterol-3-sulfate' LIPIDS. vol. 45, no. 9, 11 August 2010, pages 821 - 832, XP055162860

## Description

### FIELD OF THE INVENTION

The invention generally relates to a novel cholesterol metabolite, 5-cholesten-3β, 25-diol, disulfate (25HCDS) and uses thereof. In particular, the invention provides 25HCDS for the prevention and treatment of diseases such as lipid metabolic disorders and inflammatory disorders e.g. hyperlipidemia, diabetes, fatty liver diseases and atherosclerosis.

### BACKGROUND OF THE INVENTION

The liver plays a pivotal role in the maintenance of lipid homeostasis. Accumulation of lipids in liver tissues leads to nonalcoholic fatty liver diseases (NAFLD). NAFLD affects almost one-quarter of the general U.S. population and can progress to significant cirrhosis and hepatocellular carcinoma. The spectrum of NAFLD ranges from simple nonprogressive steatosis to progressive nonalcoholic steatohepatitis (NASH) that results in liver cirrhosis and hepatocellular carcinoma. The pathogenesis of NAFLD is viewed as a two-step process. The first step is the accumulation of triglycerides and associated lipids in the hepatocytes. The second step is the occurrence of liver inflammation. The hallmark feature of NAFLD is characterized by increased intrahepatic triglyceride accumulation. Lowering lipid levels is an important element of successful NAFLD therapy. In mammals, sterol regulatory element-binding protein-1c (SREBP-1c) preferentially controls lipogenic gene expression; and regulates fatty acid and triglyceride homeostasis. Its role in fatty acid biosynthesis and the development of fatty liver disease is well documented. However, there is currently no approved treatment for NAFLD.

Oxysterols can act at multiple points in cholesterol homeostasis and lipid metabolism. The oxysterol receptor, LXR, is sterol regulated transcription factor of lipid metabolism. Activation of LXR stimulates the expression of cholesterol efflux and clearance through ABCA1 and ABCG5/8, but it also up-regulates the expression of SREBP-1c, which in turn regulates at least 32 genes involved in lipid biosynthesis and transport. Therefore, while activation of LXR by synthetic ligands could reduce serum cholesterol level to protect against atherosclerosis, activation also leads to hepatic steatosis and hypertriglyceridemia due to the induction of fatty acid and triglyceride synthesis through activation of SREBP-1c. Hepatocytes have a limited capacity to store fatty acids in the form of triglycerides. Once the capacity is overwhelmed, cell damage occurs. Excess amounts of intracellular free fatty acids trigger the production of reactive oxygen species (ROS), causing lipotoxicity and activation of inflammatory signaling pathways, which ultimately lead to apoptosis.

5-Cholesten-3β, 25-diol 3-sulfate (25HC3S) is an oxysterol that was recently identified in primary rat hepatic nuclei. 25HC3S is disclosed in WO 2006/047022. This oxysterol may be synthesized by sterol sulfotransferase SULT2B1b from 25-hydroxycholesterol (25HC) by oxysterol sulfation Exogenous administration of a similar cholesterol metabolite, 5-cholesten-3β,25-diol 3β-sulfate (25HCβS), decreases both SREBP-1 and SREBP-2 expression; blocks the SREBP-1c processing; and represses the expression of key enzymes involved in lipid metabolism including acetyl-CoA carboxylase-1 (ACC-1), fatty acid synthase (FAS) and 3-hydroxy-3-methylglutaryl-CoA reductase (HMGR), subsequently decreasing neutral lipid and cholesterol levels.

The results indicate that 25HC3S acts as a LXR antagonist and as a cholesterol satiety signal; suppressing fatty acid and triglyceride synthetic pathway via inhibition of LXR/SREBP signaling. Moreover, 25HC3S increases IκBβ expression; blocks TNFα-induced IκBβ degradation; and decreases nuclear NFκB levels. In contrast, 25HC acts in an opposite manner, inducing IκBβ degradation and nuclear NFκB accumulation. These results indicate that 25HC3S is also involved in inflammatory responses and may represent a link between inflammatory pathways and the regulation of lipid homeostasis.

### SUMMARY OF THE INVENTION

Another regulatory cholesterol metabolite, 5-cholesten-3β, 25-diol, disulfate (25HCDS) has now been identified. Studies of 25HCDS indicate that decreased expression of this naturally occurring metabolite plays an important role in both lipid accumulation and cell injury in hepatocytes and macrophages, thereby contributing to pathogenesis of metabolic disorders. Addition of 25HCDS to the culture media of hepatocytes and macrophages decreased mRNA levels of sterol regulatory element binding proteins (SREBPs), inhibited SREBPs processing, and subsequently down-regulated key enzymes involved in lipid biosynthesis, leading to decreased intracellular lipid levels in hepatocytes and macrophages. 25HCDS also increased expression of peroxisome proliferation activator receptor (PPAR), IκB, and peroxisome proliferation activator receptor coactivator 1 alpha (PGC-1α) mRNA levels, decreased nuclear NFκB levels, and reduced pro-inflammatory cytokine expression and secretion. Significantly, *in vivo* studies showed that 25HCDS administration decreased hepatic neutral lipids by ∼20-35% without exhibiting toxicity.

Thus, the newly discovered cholesterol metabolite 25HCDS functions as an authentic PPARγ agonist and LXRs antagonist which inhibits cholesterol and lipid biosynthesis in hepatocytes and macrophages in vitro and in vivo, in addition to repressing inflammatory responses via the PPARγ/IκB/NFκB signaling pathway. 25HCDS, which has been chemically synthesized as described in the Example section herein, can thus be used as a medicament for the treatment and prevention of lipid metabolic and inflammatory disorders, including hyperlipidemia, atherosclerosis, diabetes, fatty liver diseases, etc.

Other features and advantages of the present invention will be set forth in the description of invention that follows, and in part will be apparent from the description or may be learned by practice of the invention. The invention will be realized and attained by the compositions and methods particularly pointed out in the written description and claims hereof.

In one aspect, the invention provides a compound which is: (i) 5-cholesten-3, 25-diol, disulfate (25HCDS) of the formula 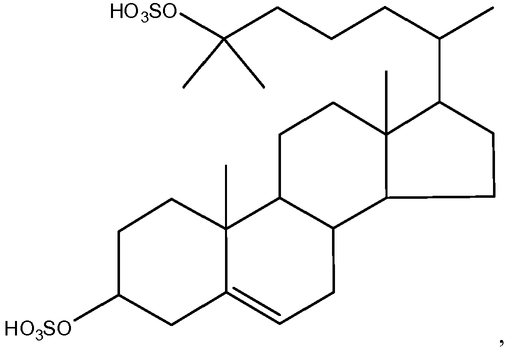 or (ii) a pharmaceutically acceptable salt thereof; for use as a medicament.
In some aspects, the compound is 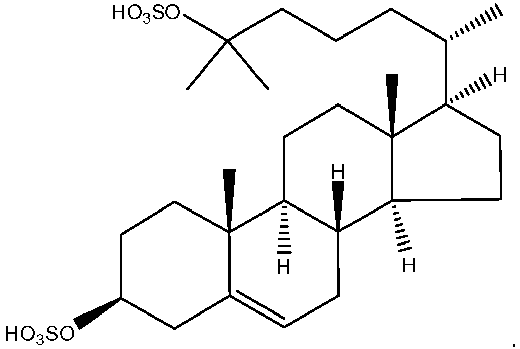 In some aspects, the invention provides the compound for use in a method of: reducing lipids in a subject in need thereof; reducing cholesterol and lipid biosynthesis in a subject in need thereof; reducing inflammation in a subject in need thereof; treating diabetes in a subject in need thereof; treating hyperlipidemia in a subject in need thereof; treating atherosclerosis in a subject in need thereof; treating fatty liver disease in a subject in need thereof; or treating inflammatory disease in a subject in need thereof.

In some aspects, the compound is administered in an amount ranging from 0.1 mg/kg to 100 mg/kg based on body mass of said subject, or the compound is administered in an amount ranging from 1 mg/kg to 10 mg/kg, based on body mass of said subject; and/or the administration comprises at least one of oral administration, enteric administration, sublingual administration, transdermal administration, intravenous administration, peritoneal administration, parenteral administration, administration by injection, subcutaneous injection and intramuscular injection.

In one aspect, the invention provides a compound which is: (i) 5-cholesten-3, 25-diol, disulfate (25HCDS) of the formula 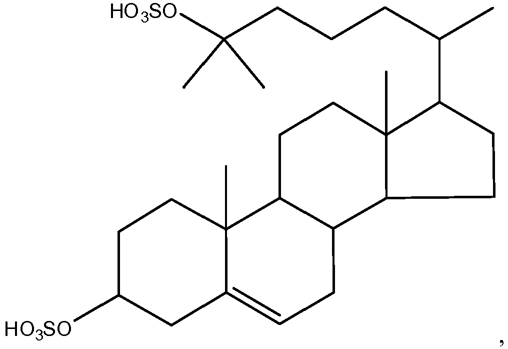 or (ii) a pharmaceutically acceptable salt thereof. In some aspects, the compound is 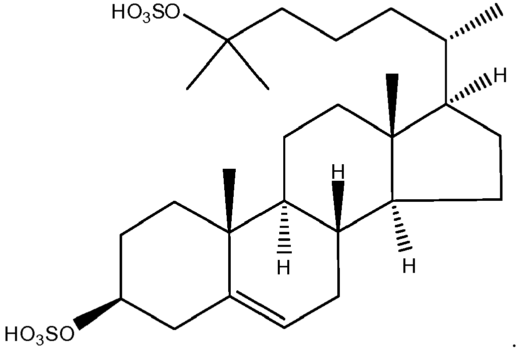 In some aspects, the compound is an isolated compound. In other aspects, the compound is substantially pure. In yet other aspects, the compound is in solid form. The solid form may be in powder form; and/or in freeze-dried form.

The invention further provides pharmaceutical compositions comprising a compound which is: (i) 5-cholesten-3, 25-diol, disulfate (25HCDS) of the formula 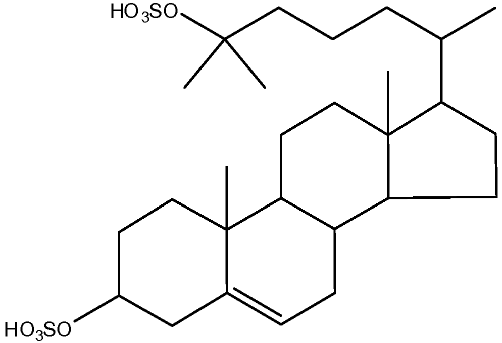 or a pharmaceutically acceptable salt thereof and (ii) a physiologically acceptable excipient, diluent or carrier.
In some aspects, the compound is 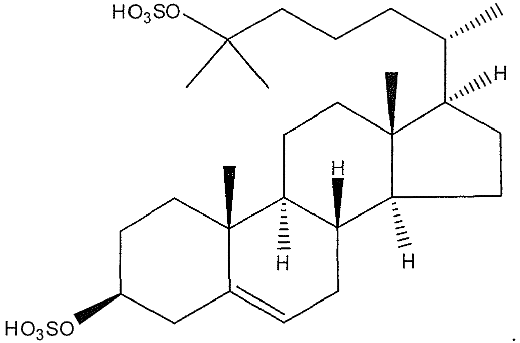 In some aspects, the pharmaceutical composition is formulated in unit dosage form. In other aspects, the pharmaceutical composition is in solid form. Solid forms of the composition include those in which: the pharmaceutical composition is in the form of a powder, a tablet, a capsule or a lozenge; or the composition comprises the compound in freeze-dried form together with a bulking agent, the composition optionally being in a sealed vial, ampoule, syringe or bag. In some aspects, the pharmaceutical composition comprises a carrier that is a liquid. In this aspect, the compound may be solubilized in the liquid or dispersed in the liquid; and/or the liquid is aqueous; and/or the liquid is sterile water for injections or phosphate-buffered saline; and/or the composition is in a sealed vial, ampoule, syringe or bag.

The invention also provides processes of producing a compound 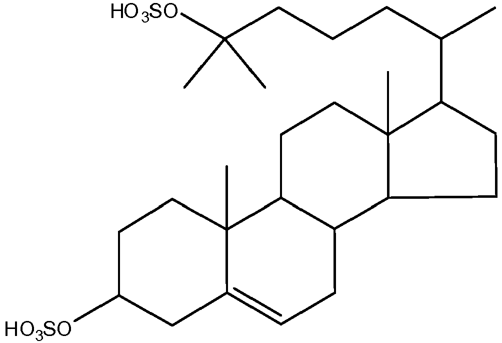 or a pharmaceutically acceptable salt thereof or a compound 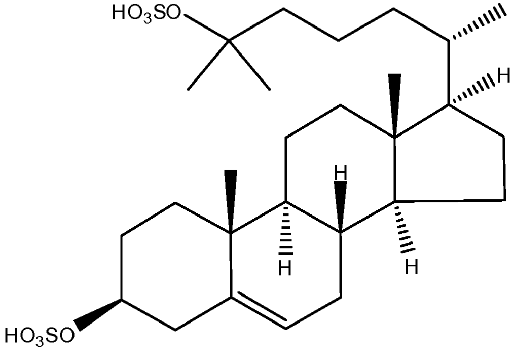 which process comprises reacting 25-hydroxycholesterol with a source of sulfur trioxide and, optionally, forming a pharmaceutically acceptable salt from the resulting 5-cholesten-3, 25-diol, disulfate (25HCDS). In some aspects, the source of sulfur trioxide is a sulfur trioxide amine complex. The invention also provides a process of producing the pharmaceutical composition, which process comprises combining the compound with a physiologically acceptable excipient, diluent or carrier.

As indicated above, the present invention *inter alia* provides the specified compounds for use in a method of: reducing lipids in a subject in need thereof; reducing cholesterol and lipid biosynthesis in a subject in need thereof; reducing inflammation in a subject in need thereof; treating diabetes in a subject in need thereof; treating hyperlipidemia in a subject in need thereof; treating atherosclerosis in a subject therefore; treating fatty liver disease in subject in need thereof; or treating inflammatory disease in a subject in need thereof. For the avoidance of doubt, in this aspect the present invention may provide the specified compound for use as a medicament in the specified method. Further, the present invention may provide the specified compound as an active therapeutic ingredient in the specified method. Further, the present invention may provide the specified compound for use in a method of treatment of the human or animal body by therapy, the method comprising the specified method.

### BRIEF DESCRIPTION OF THE DRAWINGS

**Figure 1****.** Characterization of nuclear oxysterol as 5-cholesten-3β, 25-diol disulfate by negative ion-triple quadruple mass spectrometry. HPLC/MS negative full scan spectrum, HPLC-MS elution profile sorted with mass ion 80 from product scan spectrum of m/z 583 and m/z 561 is shown.
**Figure 2****.** Analysis of chemically synthesized 25HCDS. MS spectrum of the product. 1
**Figure 3****.** H NMR spectrum of 25HCDS. The arrows indicate the proton at the 3 position of the compound and its resonance chemical shift in the starting material and in the product.
**Figure 4****.** ¹³C NMR spectrum of 25HCDS. The arrows indicate the 3 and 25 carbon positions of the compound and its resonance chemical shift in the starting material and in the product.
**Figure 5A-D****.** 25HCDS regulates lipid biosynthetic gene expression. **A**, Real time RT-PCR analysis of SREBP-1c, ACC, and FAS mRNA levels in THP-1 cells treated with 25HCDS at the indicated concentration is shown; B, SREBP-2, HMG-CoA reductase, and LDLR; PPARg and IkB mRNA levels in THP-1 cells treated with 25HCDS at indicated times, (C) and at indicated concentrations (D). The expression levels were normalized to GAPDH. Each value represents the mean of three separate measurements ± standard derivation.
**Figure 6****.** Administration of 25HCDS decreases lipid accumulation in liver tissue in mouse NAFLD models. Animals were peritoneal-injected with 25HCDS once every 3 days for 6 weeks. Hepatic triglyceride, free fatty acid, total cholesterol, free cholesterol, and cholesterol ester, free fatty acid, and triglyceride were determined as described in the Example. Each individual level was normalized by protein concentration. All the values are expressed as mean ± SD; Symbol * represents *p* < 0.05 versus HFD-fed vehicle-treated mice liver.

### DETAILED DESCRIPTION

A novel cholesterol metabolite 5-cholesten-3β,25-diol, disulfate (25HCDS), has now been identified. Administration of 25HCDS substantially increased expression of PPARγ, PPARγ coactivator 1 alpha (PGC-1α), and IκB, and decreased hepatic triglyceride and cholesterol levels via LXR-SREBP-1c signaling pathway in vivo in mouse NAFLD models. These findings demonstrate that 25HCDS is a potent regulator involved in lipid metabolism and inflammatory responses.

The invention thus provides methods of using 25HCDS for the treatment and prevention of lipid metabolic and inflammatory disorders. In some aspects, the methods involve administering a therapeutically effective dose of 25HCDS to subjects in need of such treatment, in order to elevate the level of 25HCDS in the subject and/or to effect beneficial changes in lipid metabolism. Implementation of the methods generally involves identifying patients suffering from or at risk for developing lipid metabolic disorders and conditions associated therewith, and/or identifying patients suffering from or at risk for developing abnormal inflammation, and administering 25HCDS in an acceptable form by an appropriate route. Identification of suitable subjects may be accomplished, for example, by using various blood tests, liver biopsy results, the presence of overt disease symptoms, etc., as is known in the art. Suitable subjects for treatment include those which are identified as suffering from or likely to suffer from a lipid metabolic disorder and/or inflammation. 25HCDS and related pharmaceutical compositions are also provided according to the present invention. These can be used in the treatment methods.

The 25HCDS may be in the form of a pharmaceutically acceptable salt. The pharmaceutically acceptable salt may be a di-addition salt or a mono-addition salt. A di-addition salt is formed by loss of the hydrogen atoms on each of the two sulfate groups of the 25HCDS molecule. A mono-addition salt is formed by the loss of the hydrogen atom on only one of the two sulfate groups of the 25HCDS molecule (either at the 3 or the 25-position of the molecule).

The pharmaceutically acceptable salt may, for example, be an alkali metal salt (e.g., a lithium, sodium or potassium salt), an alkaline earth metal salt (e.g., a calcium salt) or an ammonium salt. The pharmaceutically acceptable salt may, for example, be a sodium, potassium, calcium, lithium or ammonium salt.

One example of such a salt is a sodium salt of 25HCDS, for example a mono-addition sodium salt of 25HCDS, such as the mono-addition salt formed by loss of the hydrogen atom on the sulfate group at the 25-position of 25HCDS, i.e. the compound having the formula

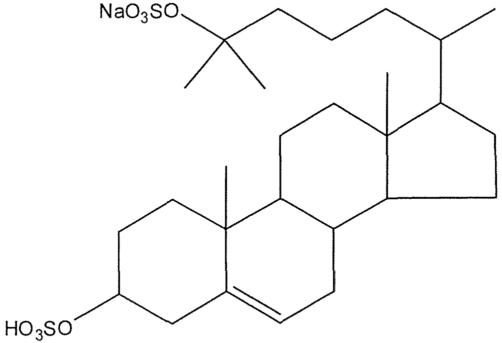

For the avoidance of doubt, it is emphasized that references throughout this specification to "25HCDS" include pharmaceutically acceptable salts of 25HCDS unless explicitly indicated otherwise.

Cholesterol contains eight chiral centers, thus giving rise to a large number of distinguishable stereoisomeric isomers. These eight chiral centers are also present in 25HCDS. In general, the 25HCDS used in the present invention may be in any single stereoisomeric form or may be a mixture of any two of more of these stereoisomeric forms. However, at least 50 wt%, preferably at least 90 wt% and most preferably at least 95 wt% of the 25HCDS may have the formula

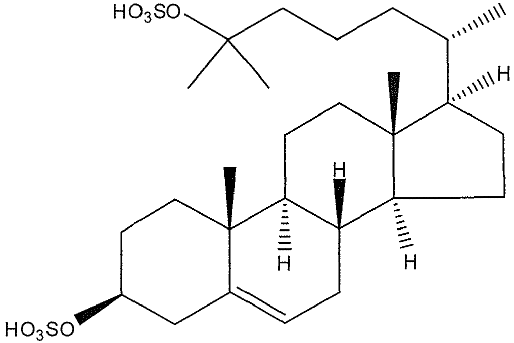

It will be appreciated that the chirality at each of the eight chiral centers in this formula is analogous to that found in native cholesterol. Thus, this stereoisomer corresponds to the stereoisomeric form of the native 25HCDS metabolite *in vivo.*

The 25HCDS or a pharmaceutically acceptable salt thereof may be isolated 25HCDS or a pharmaceutically acceptable salt thereof. "Isolated" means not comprised within tissue material contained within, or extracted from, a human or animal subject. For example, isolated 25HCDS or a pharmaceutically acceptable salt thereof is not comprised within a cell. Thus, isolated 25HCDS or a pharmaceutically acceptable salt thereof is clearly distinguishable from native 25HCDS that is comprised within tissue material (e.g., a cell) that is itself contained within, or has been extracted from, a human or animal subject.

The 25HCDS or a pharmaceutically acceptable salt thereof may be substantially pure. For example, 25HCDS or a pharmaceutically acceptable salt thereof may be provided in a substantially purified form for use in the treatment methods.

When it is "substantially pure" or "substantially purified" the disulfated oxysterol (the 25HCDS or a pharmaceutically acceptable salt thereof) may be in a form that is at least about 75%, preferably at least about 80%, more preferably at least about 90%, and most preferably at least about 95% or more free from other chemical species. Substantially pure 25HCDS or a pharmaceutically acceptable salt thereof may in particular comprise at least about 90 wt% or at least about 95%, and more preferably at least about 98 wt%, at least about 99 wt% or, even more preferably, at least about 99.5 wt% or at least about 99.8 wt% of 25HCDS or a pharmaceutically acceptable salt thereof.

The 25HCDS or a pharmaceutically acceptable salt thereof may be solid. For example, the 25HCDS or a pharmaceutically acceptable salt thereof may be in the form of a powder.

The 25HCDS or a pharmaceutically acceptable salt thereof may be in freeze-dried form. As is well-known, freeze-drying is a dehydration process typically used to preserve perishable material or make the material more convenient for transport. There are three main stages to this technique, namely freezing, primary drying and secondary drying. Freezing is typically performed using a freeze-drying machine. During primary drying the pressure is controlled by the application of appropriate levels of vacuum whilst enough heat is supplied to enable any water present to sublimate. In the secondary drying process, water of hydration is removed by the further application of heat. Typically, the pressure is also lowered to encourage further drying. After completion of the freeze-drying process, the vacuum can either be broken with an inert gas such as nitrogen prior to sealing or the material can be sealed under vacuum.

While it is possible to isolate and purify 25HCDS from living cells, those of skill in the art will recognize that in order to generate sufficient quantities of the disulfated oxysterol, the compound will generally be synthesized, either by synthetic chemical means, or by methods which involve the use of recombinant DNA technology (e.g. by using cloned enzymes to carry out suitable modifications of cholesterol). An exemplary synthesis scheme is provided in the Examples section below.

More generally, the 25HCDS or a pharmaceutically acceptable salt thereof may be produced synthetically by reacting 25-hydroxycholesterol with a source of sulfur trioxide, and, optionally, forming a pharmaceutically acceptable salt from the resulting product.

Any suitable source of sulfur trioxide may be used to convert the two hydroxyl groups (-OH) present in 25-hydroxycholesterol into sulfate groups (-OSO₃H). Sulfur trioxide-amine complexes are one exemplary group of sulfur trioxide sources. Examples of such complexes include sulfur trioxide trimethylamine complex (TMAS), sulfur trioxide triethylamine complex (TEAS), sulfur trioxide dimethylaniline complex (DMAS), sulfur trioxide dimethylformamide complex (DMFS), sulfur trioxide pyridine complex (PSS) and sulfur trioxide polyvinylpyridine complex (PVPS). Typically from one to twenty moles, for example from two to ten moles, of the chosen sulfur trioxide source (such as the sulfur trioxide-amine complex) are used per mole of 25-hydroxycholesterol.

The reaction is typically carried out in an inert solvent. The solvent may, for example, be an anhydrous solvent. One exemplary such solvent is anhydrous pyridine.

A base may also be added, for example in order to generate the desired pharmaceutically acceptable salt from the disulfate product. One such base is NaOH, which may be used in order to generate a sodium salt of 25HCDS. It will readily be appreciated that alternative reagents (having differing basicities and/or different cations) may be used to generate other pharmaceutically acceptable salts.

The reaction temperature may typically be from 10 to 100 °C, for example from 20 to 80 °C. The reaction time may typically be from 0.1 to 24 hours, for example from 0.25 to 5 hours.

If desired, the product may be purified from the reaction mixture after the reaction has taken place. If desired, the product may be isolated from the reaction mixture after the reaction has taken place

The 25-hydroxycholesterol starting material is a commercially available product. Alternatively, it may be prepared by hydroxylating cholesterol (see for example Ogawa et al. Steroids 74:81-87). The process may therefore further comprise an initial step of hydroxylating cholesterol to produce the 25-hydroxycholesterol.

25HCDS may be administered in pure form or in a pharmaceutically acceptable formulation. Such formulations (compositions) typically include 25HCDS or a pharmaceutically acceptable salt thereof and a physiologically acceptable (compatible) excipient, diluent or carrier/vehicle. The 25HCDS may be, for example, in the form of a pharmaceutically acceptable salt (e.g. an alkali metal salt such as sodium, potassium, calcium, lithium, ammonium, etc.), or other complex.

The pharmaceutical composition is sterile. Sterile means substantially free of viable microbes, for example as determined using the USP sterility test (see "The United States Pharmacopeia", 30th Revision, The United States Pharmacopeial Convention: 2008.). In order to maintain sterility, the pharmaceutical composition may be presented in a sealed package that is capable of preventing ingress of viable microbes. For example, in the case of a liquid pharmaceutical composition, the composition may be sealed in a vial or ampoule.

It should be understood that pharmaceutically acceptable formulations (compositions) include liquid and solid materials conventionally utilized to prepare both injectable dosage forms and solid dosage forms such as tablets, lozenges, powders and capsules, as well as aerosolized dosage forms. The compounds may be formulated with aqueous or oil based vehicles. Water may be used as the carrier for the preparation of compositions (e.g. injectable compositions), which may also include conventional buffers and agents to render the composition isotonic and to maintain a physiologically acceptable pH. Other potential additives (preferably those which are generally regarded as safe [GRAS]) include: colorants; flavorings; surfactants (TWEEN, oleic acid, etc.); solvents, stabilizers, elixirs, and binders or encapsulants (lactose, liposomes, etc). Solid diluents and excipients include lactose, starch, conventional disintegrating agents, coatings and the like. Preservatives such as methyl paraben or benzalkium chloride may also be used.

In further detail, when the composition is in solid form it may be in the form of a powder, a tablet, a capsule or a lozenge. When the composition is in solid form the composition may comprise the 25HCDS in freeze-dried form together with a bulking agent. A bulking agent is a pharmaceutically inactive and typically chemically inert substance that may be added to a composition to increase its bulk. Common bulking agents for use in the preparation of freeze-dried pharmaceutical compositions, and which are suitable here, include mannitol and glycine. When the composition is in solid form it may optionally be in a sealed vial, ampoule, syringe or bag.

When the pharmaceutical composition comprises a liquid carrier, the 25HCDS may be solubilized in said liquid or dispersed in said liquid; and/or the liquid may be aqueous; and/or the liquid may be sterile water for injections or phosphate-buffered saline. When the pharmaceutical composition comprises a liquid carrier, the composition may be in a sealed vial, ampoule, syringe or bag.

Depending on the formulation, it is expected that the active agent 25HCDS will consist of about 1% to about 99% by weight of the composition and the vehicular "carrier" will constitute about 1% to about 99% by weight of the composition. The pharmaceutical compositions of the present invention may include any suitable pharmaceutically acceptable additives or adjuncts to the extent that they do not hinder or interfere with the therapeutic effect of the sulfated oxysterol.

Administration may be at least one of oral administration, enteric administration, sublingual administration, transdermal administration, intravenous administration, peritoneal administration, parenteral administration, administration by injection, subcutaneous injection, and intramuscular injection. For example, administration may be oral or parenteral, including intravenously, intramuscularly, subcutaneously, intradermal injection, intraperitoneal injection, etc., or by other routes (e.g. transdermal, sublingual, oral, rectal and buccal delivery, inhalation of an aerosol, etc.). In a preferred embodiment, administration is oral. Further, administration of the compound may be carried out as a single mode of therapy, or in conjunction with other therapies, e.g. with lipid or cholesterol lowering drugs, exercise and diet regimens, etc., as described above for treatment regimens which may be undertaken by a subject upon detection of a lipid metabolic disorder. The administration of 25HCDS to a patient may be intermittent, or at a gradual or continuous, constant or controlled rate. In addition, the time of day and the number of times per day that the pharmaceutical formulation is administered may vary and are best determined by a skilled practitioner such as a physician.

The exact dosage of 25HCDS to be administered may vary depending on the age, gender, weight, overall health status of the individual patient, etc., as well as on the precise etiology of the disease. However, in general for administration in mammals (e.g. humans), therapeutically effective dosages are in the range of from about 0.1 to about 100 mg or more of compound per kg of body weight per 24 hr., and usually about 0.5 to about 50 mg of compound per kg of body weight per 24 hr., and frequently about 1 to about 10 mg of compound per kg of body weight per 24 hr., are effective.

A pharmaceutical composition of the invention may be formulated in unit dosage form, i.e., the pharmaceutical composition may be in the form of discrete portions each containing a unit dose of the 25HCDS. In this context, a unit dose may comprise, for example, from about about 0.1 mg to about 100 mg, or from about 0.5 mg to about 50 mg, or from about 1 mg to about 10 mg of 25HCDS.

The pharmaceutical composition may be prepared by combining the 25HCDS with the chosen physiologically acceptable excipients, diluents and/or carriers.

While the subjects are usually humans, veterinary applications of the technology are also contemplated.

In other aspects, the level of 25HCDS is elevated in a subject in need thereof by increasing endogenous expression/production of 25HCDS. Exemplary methods for doing so include providing the subject with one or more enzymes responsible for the synthesis of 25HCDS. In some embodiments, the enzymes themselves are provided; in other embodiments, nucleic acids which encode the enzymes are provided. The enzymes which are involved in the synthesis of 25HCDS are SULT2Bab and SULT2B1a, and one or both of these may be administered in order to elevate endogenous levels of 25HCDS. For example, vectors which contain and express one or both of these enzymes may be provided. Exemplary vectors include but are not limited to adenoviral vectors, retroviral vectors, replication-competent vectors herpes viral vectors, etc.

Lipid metabolic disorders that may be prevented or treated by elevating 25HCDS levels in a subject as described herein include but are not limited to: hepatitis (liver inflammation) caused mainly by various viruses but also by some bacterial infections, drugs or chemicals (e.g. poisons, alcohol), as well as associated complications such as liver fibrosis; autoimmunity (autoimmune hepatitis) or hereditary conditions; non-alcoholic fatty liver disease (NAFLD) a spectrum disease associated with obesity and characterized by an abundance of fat in the liver, and various syndromes associated with NAFLD (e.g. hepatitis, non-alcoholic steatohepatitis (NASH), cirrhosis, end stage liver disease, etc.); cirrhosis, i.e. the formation of fibrous scar tissue in the liver due to replacing dead liver cells (the death of liver cells can be caused, e.g. by viral hepatitis, alcoholism or contact with other liver-toxic chemicals); hemochromatosis, a hereditary disease causing the accumulation of iron in the body, eventually leading to liver damage; cancer of the liver (e.g. primary hepatocellular carcinoma or cholangiocarcinoma and metastatic cancers, usually from other parts of the gastrointestinal tract); Wilson's disease, a hereditary disease which causes the body to retain copper; primary sclerosing cholangitis, an inflammatory disease of the bile duct, likely autoimmune in nature; primary biliary cirrhosis, an autoimmune disease of small bile ducts; Budd-Chiari syndrome (obstruction of the hepatic vein); Gilbert's syndrome, a genetic disorder of bilirubin metabolism, found in about 5% of the population; glycogen storage disease type II; as well as various pediatric liver diseases, e.g. including biliary atresia, alpha-1 antitrypsin deficiency, alagille syndrome, and progressive familial intrahepatic cholestasis, etc. In addition, liver damage from trauma may also be treated, e.g. damage caused by accidents, gunshot wounds, etc. Further, liver damage caused by certain medications may be prevented or treated, for example, drugs such as the antiarrhythmic agent amiodarone, various antiviral drugs (e.g. nucleoside analogues), aspirin (rarely as part of Reye's syndrome in children), corticosteroids, methotrexate, tamoxifen, tetracycline, etc. are known to cause liver damage. In some embodiments, the diagnostic and treatment methods are performed in association with (e.g. before, during or after) liver surgery in a subject. For example, the liver surgery may be liver transplant surgery and the subject that is treated may be a donor or a recipient; or the liver surgery may be surgery that removes diseased or damaged liver tissue, or that removes cancerous tumors, etc.

In some embodiments, the disease or condition that is prevented or treated is or is caused by hyperlipidemia. By "hyperlipidemia" we mean a condition of abnormally elevated levels of any or all lipids and/or lipoproteins in the blood. Hyperlipidemia includes both primary and secondary subtypes, with primary hyperlipidemia usually being due to genetic causes (such as a mutation in a receptor protein), and secondary hyperlipidemia arising from other underlying causes such as diabetes. Lipids and lipid composites that may be elevated in a subject and lowered by the treatments described herein include but are not limited to chylomicrons, very low-density lipoproteins, intermediate-density lipoproteins, low-density lipoproteins (LDLs) and high-density lipoproteins (HDLs). In particular, elevated cholesterol (hypercholesteremia) and triglycerides (hypertriglyceridemia) are known to be risk factors for blood vessel and cardiovascular disease due to their influence on atherosclerosis. Lipid elevation may also predispose a subject to other conditions such as acute pancreatitis. The methods of the invention thus may also be used in the treatment or prophylaxis (e.g. prophylactic treatment) of conditions that are or are associated with elevated lipids. Such conditions include, for example, but are not limited to: hyperlipidemia, hypercholesterolemia, hypertriglyceridemia, fatty liver (hepatic steatosis), metabolic syndrome cardiovascular diseases, coronary heart disease, atherosclerosis (i.e. arteriosclerotic vascular disease or ASVD) and associated maladies, acute pancreatitis, various metabolic disorders, such as insulin resistance syndrome, diabetes, polycystic ovary syndrome, fatty liver disease, cachexia, obesity, arteriosclerosis, stroke, gall stones, inflammatory bowel disease, inherited metabolic disorders such as lipid storage disorders, and the like. In addition, various conditions associated with hyperlipidemia include those described in issued US patents 8,003,795 (Liu, et al) and 8,044,243 (Sharma, et al), the complete contents of both of which are herein incorporated be reference in entirety.

In some embodiments, the diseases and conditions that are prevented or treated include inflammation, and/or diseases and conditions associated with, characterized by or caused by inflammation. These include a large group of disorders which underlie many human diseases. In some embodiments, the inflammation is acute, resulting from e.g. an infection, an injury, etc. In other embodiments, the inflammation is chronic. In some embodiments, the immune system is involved with the inflammatory disorder as seen in both allergic reactions and some myopathies. However, various non-immune diseases with etiological origins in inflammatory processes may also be treated, including cancer, atherosclerosis, and ischemic heart disease, as well as others listed below.

Examples of disorders associated with abnormal inflammation which may be prevented or treated using 25HCDS include but are not limited to: acne vulgaris, asthma, various autoimmune diseases, Celiac disease, chronic prostatitis, glomerulonephritis, various hypersensitivities, inflammatory bowel diseases, pelvic inflammatory disease, reperfusion injury, rheumatoid arthritis, sarcoidosis, transplant rejection, vasculitis, and interstitial cystitis. Also included are inflammation disorders that occur as a result of the use of both legally prescribed and illicit drugs, as well as inflammation triggered by negative cognitions or the consequences thereof, e.g. caused by stress, violence, or deprivation.

In one aspect, the inflammatory disorder that is prevented or treated is an allergic reaction (type 1 hypersensitivity), the result of an inappropriate immune response that triggers inflammation. A common example is hay fever, which is caused by a hypersensitive response by skin mast cells to allergens. Severe inflammatory responses may mature into a systemic response known as anaphylaxis. Other hypersensitivity reactions (type 2 and type 3) are mediated by antibody reactions and induce inflammation by attracting leukocytes which damage surrounding tissue, and may also be treated as described herein.

In other aspects, inflammatory myopathies are prevented or treated. Such myopathies are caused by the immune system inappropriately attacking components of muscle, leading to signs of muscle inflammation. They may occur in conjunction with other immune disorders, such as systemic sclerosis, and include dermatomyositis, polymyositis, and inclusion body myositis.

In one aspect, the methods and compositions of the invention are used to prevent or treat systemic inflammation such as that which is associated with obesity. In such inflammation, the processes involved are identical to tissue inflammation, but systemic inflammation is not confined to a particular tissue but involves the endothelium and other organ systems. Systemic inflammation may be chronic, and is widely observed in obesity, where many elevated markers of inflammation are observed, including: IL-6 (interleukin-6), IL-8 (interleukin-8), IL-18 (interleukin-18), TNF-α (tumor necrosis factor-alpha), CRP (C-reactive protein), insulin, blood glucose, and leptin. Conditions or diseases associated with elevated levels of these markers may be prevented or treated as described herein. In some embodiments, the inflammation may be classified as "low-grade chronic inflammation" in which a two- to threefold increase in the systemic concentrations of cytokines such as TNF-α, IL-6, and CRP is observed. Waist circumference also correlates significantly with systemic inflammatory responses; a predominant factor in this correlation is due to the autoimmune response triggered by adiposity, whereby immune cells "mistake" fatty deposits for infectious agents such as bacteria and fungi. Systemic inflammation may also be triggered by overeating. Meals high in saturated fat, as well as meals high in calories have been associated with increases in inflammatory markers and the response may become chronic if the overeating is chronic.

Various facets of the invention are described in the Example below. However, the information provided in the Example should not be considered as limiting the scope of the invention in any way.

### EXAMPLE

### A Novel Cholesterol Metabolite, 5-Cholesten, 3β, 25-diol, disulfate (25HCDS), Decreases Lipid Biosynthesis and Suppresses Inflammatory Responses in vitro and in vivo

### INTRODUCTION

It has been shown that there is widespread dysregulation of lipid metabolism in non-alcoholic fatty liver diseases (NAFLD) and, specifically, there are major perturbations in cholesterol metabolism. The potential mechanisms by which such perturbations may lead to NAFLD via nuclear receptor signaling remain unclear. In the present study, a novel cholesterol metabolite, 5-cholesten-3β, 25-diol, disulfate (25HCDS) was identified in primary rat hepatocytes. As described herein, 25HCDS has now been chemically synthesized and its biological function has been studied. Administration of 25HCDS (25 µM) to human THP-1 macrophages and HepG2 cells, and in vivo to mouse NAFLD animal models, increased PPARγ and PPARγ coactivator 1 alpha (PGC-1α) expression and decreased expression of key proteins involved in lipid biosynthesis and pro-inflammatory responses. The administration markedly decreased hepatic lipid levels and suppressed inflammatory responses. Quantitative RT-PCR and Western blot analysis showed that 25HCDS strongly decreased SREBP-1/2 mRNA levels and suppressed expression of their responding genes including ACC, FAS, and HMG-CoA reductase, and increased IκB and decreased TNFα and ILβ mRNA levels. The results suggest that inhibition of lipid biosynthesis occurred via blocking SREBP signaling, and suppression of inflammatory responses via increasing PPARγ, PGC-1 α, and IκB expression. Analysis of lipid profiles in the liver tissues showed that administration of 25HCDS once every three days for 6 weeks significantly decreased total cholesterol, free fatty acids, and triglycerides by 30, 25, and 20%, respectively. 25HCDS is thus a potent regulator of lipid metabolism and inflammatory responses.

### MATERIALS AND METHODS

### Materials:

Cell culture reagents and supplies were purchased from GIBCO BRL (Grand Island, NY); 25-hydroxycholesterol from New England Nuclear (Boston, MA). THP-1 and HepG2 cells were obtained from American Type Culture Collection (Rockville, MD). The reagents for real time RT-PCR were from AB Applied Biosystems (Warrington WA1 4 SR, UK). The chemicals used in this research were obtained from Sigma Chemical Co. (St. Louis, MO) or Bio-Rad Laboratories (Hercules, CA). Polyclonal rabbit antibodies against SREBP1, SREBP-2 and HMG-CoA reductase were purchased from Santa Cruz Biotechnology (Santa Cruz, CA). All solvents were obtained from Fisher (Fair Lawn, NJ) unless otherwise indicated. The enhanced chemiluminescence (ECL) reagents were purchased from Amersham Biosciences (Piscataway, NJ). Testosterone and 27-hydroxycholesterol were obtained from Research Plus Inc. (Bayonne, NJ). LK6 20 x 20 cm thin layer chromatography (TLC) plates were purchased from Whatman Inc. (Clifton, NJ).

### Methods:

### Chemical synthesis of 5-cholesten-3β, 25-diol, disulfate

General procedure: 25-Hydroxycholesterol was prepared from cholesterol by the previously described method (Ogawa et al. Steroids 74:81-87). IR spectra were obtained in KBr discs on a JASCO FT-IR 460 plus spectrometer (Tokyo, Japan). ¹H and ¹³C NMR spectra were obtained on a Varian 500 Inova (AS500) instrument at 499.62 MHz and 125.64 MHz, respectively. Flow injection low-resolution mass (LR-MS) spectra were recorded by a Thermo Scientific TSQ Quantum Ultra MS equipped with electrospray ionization (ESI) probe under negative ion mode. High resolution mass (HR-MS) spectra were measured using Thermo Scientific LTQ Qrbitrap Discovery MS with ESI probe under the negative ion mode. Reversed-phase TLC was carried out on pre-coated RP-18F254S plates using methanol-water-acetic acid mixtures (90:10:1, v/v/v) as the developing solvent. The spots were visualized by 50% H₂SO₄ with heating at 110°C. A Bond Elute C18 cartridge (10 g; Varian,) was used for sample purification. OXONE^{®} (potassium peroxymonosulfate) and acetone were purchased from Sigma-Aldrich Co. (St. Louis, MO, USA), and all other reagents used were the highest grade except for the organic solvents which were HPLC grade.

Synthesis of 5-cholesten-3β,25-diol, disulfate (25HCDS): To a solution of 25-hydroxycholesterol (30 mg, 0.07 mmol) in anhydrous pyridine (300µL), sulfur trioxide-trimethylamine complex (45 mg) was added, and the suspension was stirred at 50°C for 1h. To the reaction mixture, 0.1N methanolic NaOH (100µL) was added and the mixture was applied to a Sep Pak C18 cartridge, which had been primed with methanol (10mL) and water (10 mL). The cartridge was successively washed with PBS (25mL) and water (25mL), and then the retained 25HCDS was eluted with 60% methanol (10ml). After 10X dilution with acetonitrile, the solvents were evaporated to dryness under N₂ stream below 40°C, and the 25HCDS was obtained in powdered form. Yield, 25 mg (60%).

### Cell culture

Human THP-1 monocytes and HepG2 cells were purchased from the American Type Culture Collection (ATCC, Manassas, VA) and maintained according to the supplier's protocols. THP-1 monocytes were differentiated to macrophages by adding 100 nM of phorbol 12-myristate 13-acetate (PMA). When cells reached ∼90% confluence, 25HCDS in ethanol (the final concentration of ethanol in media was 0.1%) was added. The cells were harvested at the indicated times for protein, mRNA, and lipid analysis.

For the study of HMG CoA expression regulation, HepG2 or PHH were cultured in the media as described above in the presence or absence of mevinolin (50 µM) and mevalonate (0.5 µM). After culturing for 48 hrs, oxysterols were added and cultured for another 6 hrs, and then the cells were harvested for determining mRNA and protein levels.

### Determination of cholesterol biosynthesis by TLC and HPLC

After incubation of THP-1 macrophages or HepG2 cells in media containing different concentrations of 25HCDS as indicated for 6 hrs, cells in 60 mm dishes were given 3 ml of the same fresh medium containing 5 µCi of [1-¹⁴C] acetate. After 2 hr incubation at 37°C, the media was removed and the cells were washed twice with phosphate-buffered saline (PBS), harvested with rubber policeman as described, and collected in microcentrifuge tubes. The cells were sedimented by centrifugation and the pellets were washed three times by resuspension and sedimentation. Subcellular fractions (microsomal, cytosol, and nuclear) were isolated as previously described (2). The cellular or subcellular pellets were resuspended in 0.3 ml of PBS. To each sample, 1.5 µg of testosterone was added as an internal standard. The total lipids were extracted and separated by adding 3 volumes of chloroform:methanol (1:1). [¹⁴C] cholesterol and hydroxycholesterols were isolated into chloroform phase and separated on TLC (toluene:acetyl acetate, 2/3, v/v/). [1-¹⁴C] acetate derivatives were visualized by Image Reader, Fujifilm BAS-1800 II as previously described (1).

For analysis of unlabeled sterol products, the extracted lipids were incubated with 2 units of cholesterol oxidase at 37°C for 20 min. The oxidation reaction was terminated by adding 1.5 ml of methanol followed by 0.5 ml of saturated KC1. The sterols were extracted twice using 3 ml of hexane. The hexane phase was collected and evaporated under a stream of nitrogen. The residues were dissolved in mobile phase solvents for HPLC analysis as previously described (3).

[1-¹⁴C]Acetate derivatives in the chloroform phase were analyzed by HPLC on an a silica column (5µ x 4.6 mm x 25 cm; Beckman, USA) using HP Series 1100 solvent delivery system (Hewlett Packard) at 1.3 ml/min flow rate. The column was equilibrated and run in a solvent system of hexane:isopropanol:glacial acetic acid (965:25:10, v/v/v), as the mobile phase. The effluents were collected every 0.5 min (0.65 ml per fraction) except as indicated. The counts in [¹⁴C] acetate derivatives were determined by Scintillation Counting. The column was calibrated with [¹⁴C] cholesterol, [³H] 25-hydroxycholesterol, and [¹⁴C] 27-hydroxycholesterol.

### Determination of mRNA levels by real-time RT-PCR

Total RNA was isolated with SV Total RNA Isolation Kit (Promega, Madison, WI), which included DNase treatment. Total RNA, 2 µg, was used for the first-strand cDNA synthesis as recommended by the manufacturer (Invitrogen, Carlsbad, CA). Real-time RT-PCR was performed using a suitable dye as indicator on ABI 7500 Fast Real-Time PCR System (Applied Biosystems, Foster City, CA). All primer/probe sets for real-time PCR were TaqMan gene expression assays (Applied Biosystems, Foster City, CA). Amplifications of β-actin and glyceraldehyde 3-phosphate dehydrogenase (GAPDH) were used as internal controls. Relative messenger RNA (mRNA) expression was quantified with the comparative cycle threshold (Ct) method and was expressed as 2^{-ΔΔCt}. The sequences of suitable primers for amplification are described, for example, in Ren et al., 2007 (1).

### Western blot analysis

Microsomal fractions were isolated as previously described (4). Microsomal or total extracted proteins from the treated cells were separated on a 7.5% SDS-polyacrylamide denaturing gel. Following SDS-PAGE, proteins were electrophoretically transferred to polyvinylidene fluoride (PVDF) membranes (Millipore). The membranes were then blocked at 25°C for 60 minutes in blocking buffer [PBS, pH 7.4, 0.1% TWEEN® 20 (membrane protein solubilizing non-ionic surfactant, C₅₈H₁₁₄O₂₆), 5% non-fat dry milk). Proteins were then incubated at 4°C for overnight with a rabbit polyclonal IgG against human SREBP1, SREBP-2, or HMG-CoA reductase. After washing with PBS, pH 7.4, containing 0.05% of TWEEN® 20, goat anti-rabbit IgG-horse-radish peroxidase conjugate, 1:2500, in washing solution was added and incubated for 60 minutes. Protein bands were detected using the Amersham ECL plus Kit. Positive bands were quantitated by the Advanced Image Data Analyzer (Aida Inc., Straubenhardt, Germany).

### Animal studies

Animal studies were approved by Institutional Animal Care and Use Committee of McGuire Veterans Affairs Medical Center and were conducted in accordance with the Declaration of Helsinki, the Guide for the Care and Use of Laboratory Animals, and all applicable regulations. To examine the effect of 25HCDS on diet-induced lipid accumulation in sera and liver, 8-week-old female C57BL/6J mice (Charles River, Wilmington, MA) were fed high fat diet (HFD) (Harlan Teklad, Madison, WI) containing 42% kcal from fat, 43% kcal from carbohydrate, 15% kcal from protein and 0.2% cholesterol for 10 weeks. All mice were housed under identical conditions in an aseptic facility and given free access to water and food. At the end of each period, the mice were intraperitoneally injected with vehicle solution (ethanol/PBS; Vehicle), or 25HCDS (25 mg/kg) once every three days for 6 weeks and fasted for overnight; and blood samples were collected. Serum triglyceride, total cholesterol, high density lipoprotein-cholesterol, glucose, alkaline phosphatase (ALK), alanine aminotransferase (ALT), and aspartate aminotransferase (AST) were measured using standard enzymatic techniques in the clinical laboratory at McGuire Veterans Affairs Medical Center. Lipoprotein profiles in sera were analyzed by HPLC as described below.

### Quantification of hepatic lipids

Liver tissues were homogenized, and lipids were extracted with a mixture of chloroform and methanol (2:1), and filtered. The extracts, 0.2 ml, were evaporated to dryness and dissolved in 100 µl of isopropanol containing 10% of TRITON™ X-100 (C₁₄H₂₂O(C₂H₄O)n), a nonionic surfactant) for cholesterol assay (Wako Chemicals USA, Richmond, VA), the NEFA solution (0.5 g of EDTA-Na₂, 2 g of TRITON™ X-100, 0.76 ml of 1N NaOH, and 0.5 g of sodium azide/1, pH 6.5) for free fatty acid assay (Wako Chemicals USA, Richmond, VA), or isopropanol only for triglyceride assay (Fisher Scientific, Pittsburgh, PA). All of the assays were performed according to the manufacturer's instructions, respectively. Each lipid concentration was normalized to liver weight.

### Statistics

Data are reported as the mean ± standard deviation. Where indicated, data were subjected to *t*-test analysis and determined to be significantly different if p<0.05.

### RESULTS

### Detection of novel cholesterol metabolite in nuclei of primary rat hepatocytes

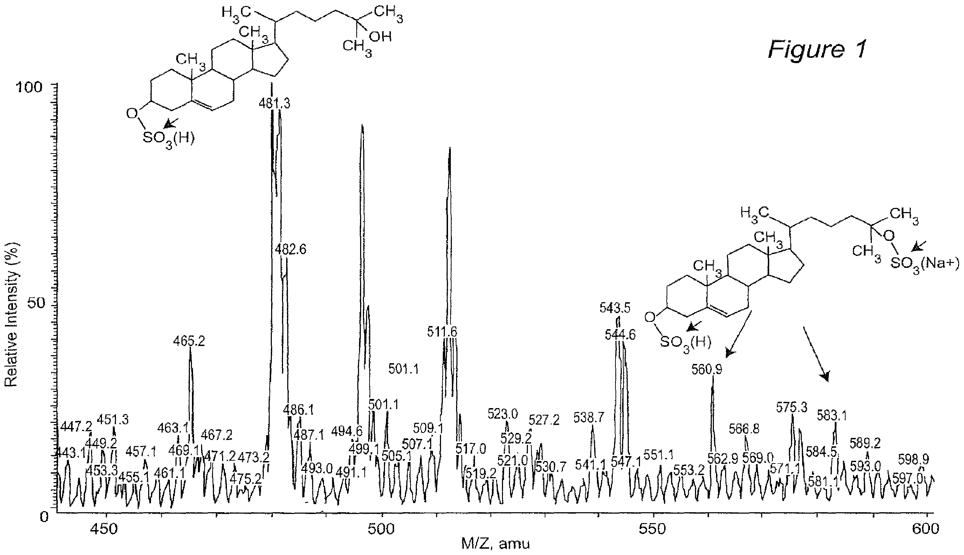

To determine the presence of new cholesterol metabolites in hepatic nuclei, nuclear fractions were isolated from primary rat hepatocytes. The oxysterols in the methanol/water phases of each fraction were analyzed by LC-MS. The results show that two of the major molecular ions, m/z 561 and m/z 583 (561+Na) are well fit to the molecule, 5-cholesten-3β, 25-diol disulfate (Figure 1). The molecule is most likely synthesized by SULT2B1b and SULT2B1a.

### Chemical synthesis of the nuclear oxysterol, 5-cholesten-3β, 25-diol, disulfate

To confirm its structure and study its role in cellular lipid homeostasis and inflammatory responses, 25HCDS was chemically synthesized as described above and purified.

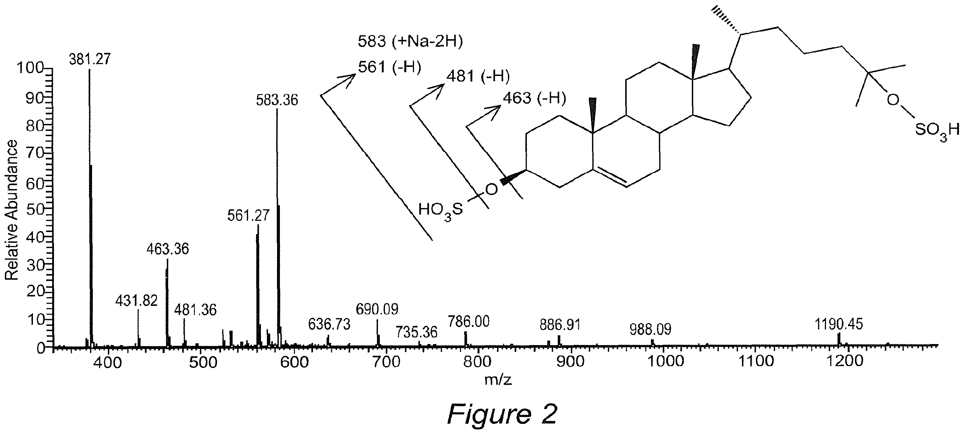

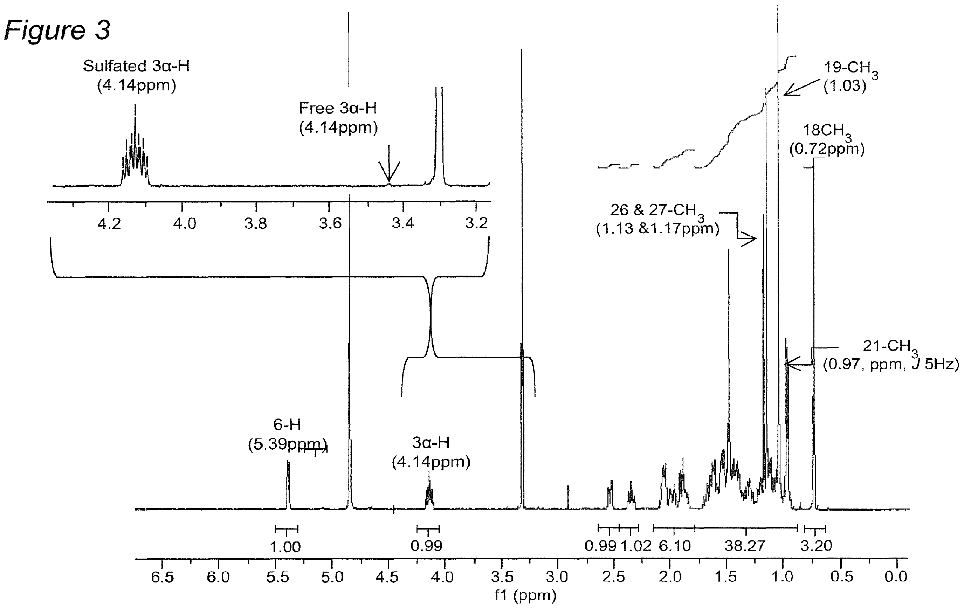

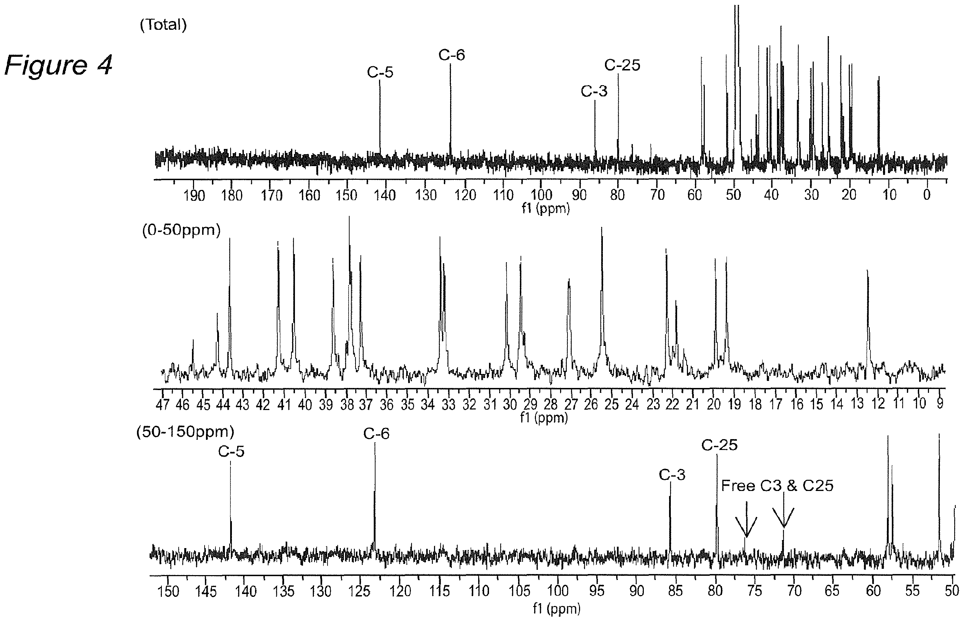

MS analysis of the synthesized compound shows the same molecular mass ion, m/z 561 and m/z 583 (+Na) as the authentic nuclear oxysterol, and the purified product was not contaminated by the starting material, 25-hydroxycholesterol, m/z 401. LR-MS (ESI-negative), m/z: 583.4 (M+Na-2H, 88%), 561.3 (M-H, 46%), 481.4 (M-SO₃-H, 11%), 463.4 (M-H₂SO₄-H, 34%), 431.82 (14%), 381.27 (100%) **(****Figure 2****).** ¹H NMR (CD₃OD) δ: 0.72 (3H, s, 18-CH₃), 0.97 (3H, d, *J* 5.0Hz, 21-CH₃), 1.03 (3H, s, 19-CH₃), 1.14 (6H, s, 26- and 27-CH₃), 4.14 (1H, br. m, 3α-H), 5.39 (1H, br. s, 6-H) **(****Figure 3****).** ¹³C NMR (CD₃OD) δ: 12.45, 19.37, 19.90, 21.82, 22.29, 25.45, 25.51, 27.05, 27.12, 29.39, 29.44, 30.13, 33.16, 33.37, 37.26, 37.32, 37.50, 38.60, 40.52, 41.27, 43.65, 51.78, 57.71, 58.37, 79.98, 85.93, 123.44, 141.71 **(****Figure 4****).** The results indicate that the synthesized molecule is 5-cholesten-3β, 25-diol, disulphate (25HCDS), and that it "fits" the indicated molecule in the hepatocyte nuclear fraction.

### 25HCDS inhibits lipid biosynthesis by decreasing ACC, FAS, and HMG-CoA reductase mRNA levels via SREBP signaling

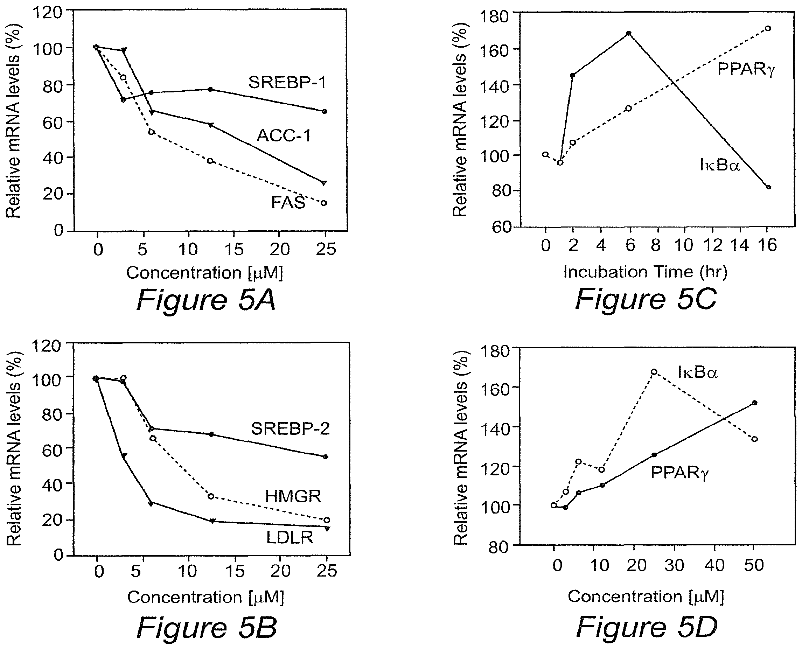

To investigate how 25HCDS inhibits lipid biosynthesis, total RNA was isolated from treated THP-1 macrophages. The mRNA levels of ACC and FAS for triglyceride synthesis, and HMG-CoA reductase for cholesterol synthesis in macrophages and hepG2 cells were determined by real time RT-PCR. As shown in Figure 5, decreases in ACC and FAS (Figure 5A), and HMG-CoA reductase mRNA levels (Figure 5B) following the addition of 25HCDS to the cells in culture were concentration dependent as shown and time dependent (data not shown). These decreases were consistent with the decreases in expression of SREBP1/2 shown in Figure 5A and 5B. These results indicate that 25HCDS decreases SREBP signaling and subsequently decreases lipid biosynthesis. Interestingly, 25HCDS linearly increased PPARγ mRNA levels and coincidently increased IκBα expression at an early stage and at low concentrations. The results suggest that 25HCDS suppress inflammatory responses via the PPARγ/IκBα signaling pathway as does 25HC3S.

### Effects of administration of 25HCDS on lipid homeostasis in HFD-fed mice

To study the effects of long-term treatment of 25HCDS on lipid homeostasis, 8-week-old C57BL/6J female mice were fed a HFD for 10 weeks, and then divided into two groups. One group was treated with 25HCDS and the other with vehicle by peritoneal injection once every three days for six weeks. During the treatment, the mice were fed a HFD, and body mass and caloric intakes were monitored. No significant difference in these two parameters was observed (data not shown). After 6 weeks of injections, the mice were fasted overnight, and sacrificed. Liver weights of the mice did not show significant differences regardless of diet (data not shown).

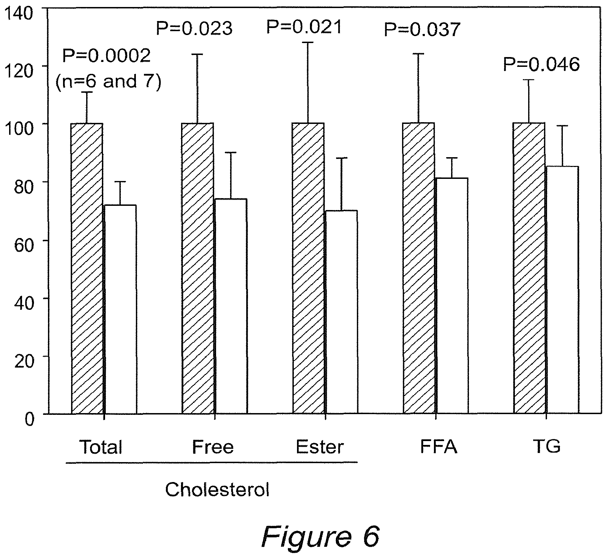

To study the effect of 25HCDS on hepatic lipid metabolism, hepatic lipid levels and related gene expression levels were determined. As previously reported, HFD-fed mice displayed increased triglyceride, total cholesterol, free fatty acid, and triglyceride levels in liver when compared to chow-fed mice (data not shown). These increases were significantly reduced by 25HCDS administration, e.g. by 30%, 20% and 18% (p < 0.05), respectively, as shown Figure 6. In addition, gene expression analysis showed that 25HCDS administration significantly decreased the expression of key enzymes and receptors involved in free fatty acid, triglyceride, and cholesterol synthesis, as shown in Table 1.

Dysregulation of lipid metabolism is frequently associated with inflammatory conditions. 25HCDS treatment significantly suppressed the expression of TNFα, and IL1β, by 50%, 36%, respectively (Table 2). These results are consistent with liver function assays which showed that 25HC3S suppresses liver inflammatory responses, decreasing liver damage and alkaline phosphatase activity in sera (data not shown). Interestingly, 25HCDS increased expression of PGC-1α by 2-fold in the liver. Thus, 25HCDS appears to regulate lipid metabolism and inflammatory responses via LXR, PPARγ and PGC-1α signaling.

**Table 1. Relative Hepatic mRNA Expression involved in lipid metabolism in the Mice Fed a HFD with or without 25HCDS**

| Gene Name | Gene description | HFD (n=6) | HFD + 25HCDS (n=7 |
|---|---|---|---|
| Fatty acid biosynthesis | | | |
| SREBP-1c | Sterol regulatory element-binding protein-1c | 1.0 ± 0.36 | 0.64 ± 0.14 * |
| ACC1 | Acetyl-CoA carboxylase 1 | 1.0 ± 0.31 | 0.86 ± 0.18 |
| FAS | Fatty acid synthase | 1.0 ± 0.27 | 0.68 ± 0.17 * |

| Triglyceride metabolism | | | |
|---|---|---|---|
| GPAM | Glycerol-3 -phosphate acyltransferase | 1.0 ± 0.10 | 0.74 ± 0.18 * |
| MTTP | Microsomal triglyceride transfer protein | 1.0 ± 0.11 | 0.94 ± 0.17 |
| PLTP | Phospholipid transfer protein | 1.0 ± 0.3 | 0.68 ± 0.21 * |

| Cholesterol Metabolism | | | |
|---|---|---|---|
| SREBP-2 | Sterol regulatory element-binding protein-2 | 1.0 ± 0.18 | 1.12 ± 0.25 |
| HMGR | Hydroxy-methylglutaryl-coenzyme A reductase | 1.0 ± 0.16 | 0.84 ± 0.07 * |
| LDLR | Low density lipoprotein receptor | 1.0 ± 0.43 | 0.62 ± 0.08 * |
| CD36 | Thrombospondin receptor | 1.0 ± 0.52 | 0.69 ± 0.29 |

| | | | |
|---|---|---|---|
| Animals were treated as described above. All values are expressed as the mean ± SD; n = 6-7. * p < 0.05 compared with HFD mice. Abbreviations: HFD, high fat diet. | | | |

**Table 2. Relative Hepatic mRNA Expression involved in inflammatory responses in the Mice Fed a HFD with or without 25HCDS**

| Gene Name | Gene description | HFD (n=6) | HFD + 25HCDS (n=7) |
|---|---|---|---|
| PGC-1α | Peroxisome proliferator-activated receptor gamma coactivator-1a | 1.0 ± 0.27 | 2.11 ± 0.82* |
| PPARα | Peroxisome proliferator-activated receptor gamma | 1.0 ± 0.42 | 1.27 ± 0.52 |
| IkBα | Nuclear factor of kappa light polypeptide gene enhancer in B-cells inhibitor a | 1.0 ± 0.25 | 1.35 ± 0.27 * |
| TNFVv | Tumor necrosis factor a | 1.0 ± 0.28 | 0.50 ± 0.21 ** |
| IL1α | Interleukin 1a | 1.0 ± 0.35 | 1.02 ± 0.20 |
| IL1β | Interleukin 1b | 1.0 ± 0.21 | 0.64 ± 0.16 ** |

| | | | |
|---|---|---|---|
| Animals were treated as described above. All values are expressed as the mean ± SD; * p < 0.05, ** p < 0.01 compared with HFD mice. Abbreviations: HFD, high fat diet. | | | |

### DISCUSSION

Cholesterol and triglyceride metabolism are closely associated. Orphan nuclear receptors are ligand-activated transcription factors that regulate the expression of key target genes which are important regulators of many biological events. The receptors for fatty acids (PPARs), oxysterols (LXRs), retinoic acids (RXR), and SREBPs function as sensors of cellular lipid levels, eliciting gene expression changes in order to maintain lipid homeostasis and protecting cells from damage by lipid accumulation. However, cross-talk among the receptor activities remains obscure. As shown herein, the cholesterol metabolite, 25HCDS, inhibits SREBP-1c expression, processing, and activity in vitro and in vivo and increases PPARγ and PGC-1α expression. It is well-documented that SREBPs control lipid biosynthesis, PPARγ regulates inflammatory responses, and PGC-1α controls energy homeostasis. Thus, the results show that 25HCDS is a potent regulator of these processes, and plays an important role in maintenance of hepatic lipid homeostasis and inflammatory responses. Administration of 25HC3S increases nuclear PPARγ protein levels and suppresses inflammatory responses but only slightly increases PPARγ mRNA. In contrast, 25HCDS significantly increases PPARγ and PGC-1α mRNA expression, in atime and concentration dependent manner, indicating 25HCDS is more potent than 25HC3S in regulation of lipid metabolism and inflammatory responses.

The reactions of 25HCDS biosynthesis and oxysterol sulfation represent a novel regulatory pathway, which mediates nuclear receptor activity in hepatocytes. Key components of this pathway are summarized as follows: **1)** when intracellular cholesterol levels are increased, mitochondrial cholesterol delivery protein, StAR, delivers cholesterol into mitochondria, where regulatory oxysterols, such as 25HC, are synthesized by CYP27A1. These oxysterols in turn activate LXR, and subsequently up-regulate expression of its target genes involved in fatty acid and triglyceride biosynthesis. In addition, 25HC activates LXR, down regulates newly synthesized cholesterol synthesis by inhibiting HMGR expression and increases ABCA1 mediated cholesterol secretion from the cells (HDL formation). **2)** 25HC3S and 25HCDS inactivate LXRs and suppress SREBP-1c processing, indicating that these sulfated oxysterols decrease intracellular lipid levels by inhibiting synthesis; **3)** the effects of 25HC on lipid metabolism are opposite to those of 25HC3S and 25HCDS. Thus, intracellular oxysterol sulfation represents a novel regulatory mechanism involved in lipid metabolism, and in the development of NAFLD.

Treatment of mouse NAFLD models with 25HCDS decreased hepatic lipid levels. A large number of treatments for NAFLD have been studied. While many appear to improve biochemical markers such as alanine transaminase levels, most have not been shown to reverse histological abnormalities or reduce clinical endpoints. 25HCDS suppresses key gene expressions involved in lipid biosyntheis at the transcriptional level via blocking activation of nuclear receptor LXRs and SREBPs, suppressing proinflammatory cytokines induced by HFD and controlling energy homeostasis via PGC1a. Thus, 25HCDS serves as a potent regulator to reduce hepatic lipid levels effectively and accordingly represents a new agent for therapy of NALFD and other lipid metabolic associated diseases.

### REFERENCES

1. Ren,S., Li,X., Rodriguez-Agudo,D., Gil,G., Hylemon,P., and Pandak,W.M. 2007. Sulfated oxysterol, 25HC3S, is a potent regulator of lipid metabolism in human hepatocytes. Biochem. Biophys. Res. Commun. 360:802-808.
2. Ren,S., Hylemon, P., Zhang,Z.P., Rodriguez-Agudo,D., Marques,D., Li,X., Zhou,H., Gil,G., and Pandak,W.M. 2006. Identification of a novel sulfonated oxysterol, 5-cholesten-3beta, 25-diol 3-sulfonate, in hepatocyte nuclei and mitochondria. J. Lipid Res. 47:1081-1090.
3. Pandak,W.M., Ren,S., Marques,D., Hall,E., Redford,K., Mallonee,D., Bohdan,P., Heuman,D., Gil,G., and Hylemon,P. 2002. Transport of cholesterol into mitochondria is rate-limiting for bile acid synthesis via the alternative pathway in primary rat hepatocytes. J. Biol. Chem. 277:48158-48164.
4. Ren,S., Hylemon,P., Marques,D., Hall,E., Redford,K., Gil,G., and Pandak,W.M. 2004. Effect of increasing the expression of cholesterol transporters (StAR, MLN64, and SCP-2) on bile acid synthesis. J. Lipid Res. 45:2123-2131.

## Claims

1. A compound which is: (i) 5-cholesten-3, 25-diol, disulfate (25HCDS) of the formula 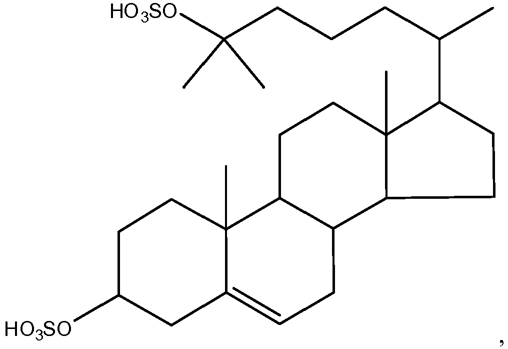 or (ii) a pharmaceutically acceptable salt thereof;
for use as a medicament.

2. The compound for use according to claim 1, wherein the compound is

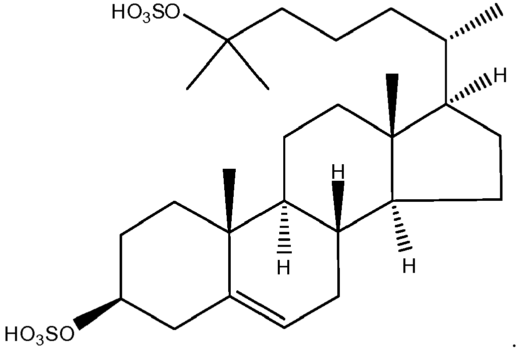

3. A compound as defined in claim 1 or 2 for use in a method of: reducing lipids in a subject in need thereof; reducing cholesterol and lipid biosynthesis in a subject in need thereof; reducing inflammation in a subject in need thereof; treating diabetes in a subject in need thereof; treating hyperlipidemia in a subject in need thereof; treating atherosclerosis in a subject in need thereof; treating fatty liver disease in a subject in need thereof; or treating inflammatory disease in a subject in need thereof.

4. The compound for use as defined in claim 3, wherein the method comprises:
- administering the compound in an amount ranging from 0.1 mg/kg to 100 mg/kg based on body mass of the subject, or administering the compound in an amount ranging from 1 mg/kg to 10 mg/kg, based on body mass of the subject; and/or
- administering the compound by at least one of oral administration, enteric administration, sublingual administration, transdermal administration, intravenous administration, peritoneal administration, parenteral administration, administration by injection, subcutaneous injection and intramuscular injection.

5. A compound as defined in claim 1 or 2.

6. The compound according to claim 5, which is an isolated compound.

7. The compound according to claim 5 or 6, which is substantially pure.

8. The compound according to any one of claims 5 to 7, which is in solid form.

9. The compound according to claim 8, which is:
- in powder form; and/or
- in freeze-dried form.

10. A pharmaceutical composition comprising: (i) a compound as defined in claim 1 or 2; and (ii) a physiologically acceptable excipient, diluent or carrier.

11. The pharmaceutical composition according to claim 10, wherein the composition is formulated in unit dosage form.

12. The pharmaceutical composition according to claim 10 or 11, wherein:
- the composition is in solid form; or
- the composition is in solid form and the composition is in the form of a powder, a tablet, a capsule or a lozenge; or
- the composition is in solid form and the composition comprises the compound in freeze-dried form together with a bulking agent, the composition optionally being in a sealed vial, ampoule, syringe or bag.

13. A pharmaceutical composition according to claim 10 or 11, which comprises a carrier that is a liquid, and optionally wherein:
- the compound is solubilized in said liquid or dispersed in said liquid; and/or
- said liquid is aqueous; and/or
- said liquid is sterile water for injections or phosphate-buffered saline; and/or
- said composition is in a sealed vial, ampoule, syringe or bag.

14. A process of producing a compound as defined in claim 1 or 2, which process comprises reacting 25-hydroxycholesterol with a source of sulfur trioxide and, optionally, forming a pharmaceutically acceptable salt from the resulting 5-cholesten-3, 25-diol, disulfate (25HCDS), wherein the source of sulfur trioxide is optionally a sulfur trioxide amine complex.

15. A process of producing a pharmaceutical composition as defined in any one of claims 10 to 13, which process comprises combining said compound with said physiologically acceptable excipient, diluent or carrier.

## Patentansprüche

1. Verbindung, die Folgendes ist: (i) 5-Cholesten-3, 25-Diol, Disulfat (25HCDS) der Formel 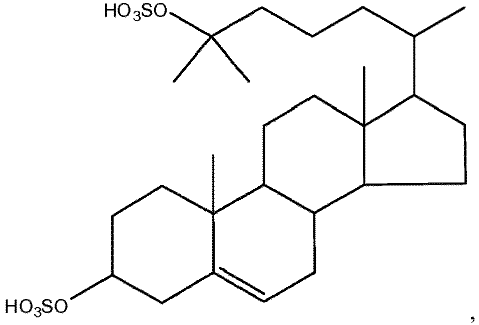 oder (ii) ein pharmazeutisch akzeptables Salz davon,
zur Verwendung als ein Medikament.

2. Verbindung zur Verwendung nach Anspruch 1, wobei die Verbindung 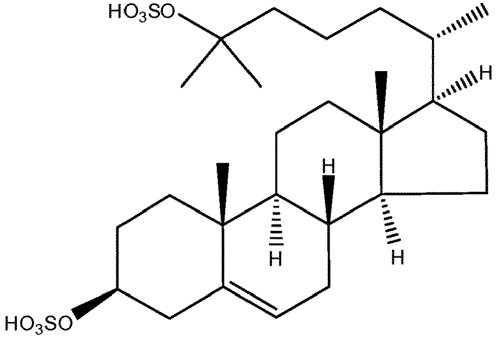 ist.

3. Verbindung nach Anspruch 1 oder 2 zur Verwendung in einem Verfahren zum: Reduzieren von Lipiden in einem Subjekt, das dies benötigt, Reduzieren von Cholesterin und Lipidbiosynthese in einem Subjekt, das dies benötigt; Reduzieren von Entzündung in einem Subjekt, das dies benötigt; Behandeln von Diabetes in einem Subjekt, das dies benötigt; Behandeln von Hyperlipidämie in einem Subjekt, das dies benötigt; Behandeln von Atherosklerose in einem Subjekt, das dies benötigt; Behandeln von Fettlebererkrankung in einem Subjekt, das dies benötigt; oder Behandeln von Entzündungserkrankung in einem Subjekt, das dies benötigt.

4. Verbindung zur Verwendung nach Anspruch 3, wobei das Verfahren umfasst:
- Verabreichen der Verbindung in einer Menge von 0,1 mg/kg bis 100 mg/kg basierend auf der Körpermasse des Subjekts, oder Verabreichen der Verbindung in einer Menge von 1 mg/kg bis 10 mg/kg, basierend auf der Körpermasse des Subjekts; und/oder
- Verabreichen der Verbindung durch mindestens eines von oraler Verabreichung, enteraler Verabreichung, sublingualer Verabreichung, transdermaler Verabreichung, intravenöser Verabreichung, peritonealer Verabreichung, parenteraler Verabreichung, Verabreichung durch Injektion, subkutaner Injektion und intramuskulärer Injektion.

5. Verbindung nach Anspruch 1 oder 2.

6. Verbindung nach Anspruch 5, die eine isolierte Verbindung ist.

7. Verbindung nach Anspruch 5 oder 6, die im Wesentlichen rein ist.

8. Verbindung nach einem der Ansprüche 5 bis 7, die in fester Form vorliegt.

9. Verbindung nach Anspruch 8, die in folgender Form vorliegt:
- in Pulverform; und/oder
- in gefriergetrockneter Form.

10. Pharmazeutische Zusammensetzung, umfassend: (i) eine Verbindung nach Anspruch 1 oder 2; und (ii) einen physiologisch akzeptablen Hilfsstoff, Verdünner oder Trägerstoff.

11. Pharmazeutische Zusammensetzung nach Anspruch 10, wobei die Zusammensetzung in einer Einzeldosisform formuliert ist.

12. Pharmazeutische Zusammensetzung nach Anspruch 10 oder 11, wobei:
- die Zusammensetzung in fester Form vorliegt; oder
- die Zusammensetzung in fester Form vorliegt und die Zusammensetzung in Form eines Pulvers, einer Tablette, einer Kapsel oder einer Pastille vorliegt; oder
- die Zusammensetzung in fester Form vorliegt und die Zusammensetzung die Verbindung in gefriergetrockneter Form zusammen mit einem Füllstoff umfasst, wobei die Zusammensetzung sich optional in einer verschlossenen Durchstechflasche, Ampulle, Spritze oder einem Beutel befindet.

13. Pharmazeutische Zusammensetzung nach Anspruch 10 oder 11, die einen Trägerstoff umfasst, der eine Flüssigkeit ist, und wobei optional:
- die Verbindung in der Flüssigkeit löslich gemacht oder in der Flüssigkeit dispergiert ist; und/oder
- die Flüssigkeit wässrig ist; und/oder
- die Flüssigkeit steriles Wasser für Injektionszwecke oder phosphatgepufferte Kochsalzlösung ist; und/oder
- die Zusammensetzung sich in einer verschlossenen Durchstechflasche, Ampulle, Spritze oder einem Beutel befindet.

14. Verfahren zum Herstellen einer Verbindung nach Anspruch 1 oder 2, wobei das Verfahren ein Reagieren von 25-Hydroxycholesterol mit einer Schwefeltrioxidquelle und optional ein Bilden eines pharmazeutisch akzeptablen Salzes von dem resultierenden 5-Cholesten-3, 25-Diol, Disulfat (25HCDS) umfasst, wobei die Schwefeltrioxidquelle optional ein Schwefeltrioxidaminkomplex ist.

15. Verfahren zum Herstellen einer pharmazeutisch akzeptablen Zusammensetzung nach einem der Ansprüche 10 bis 13, wobei das Verfahren ein Kombinieren der Verbindung mit dem physiologisch akzeptablen Hilfsstoff, Verdünner oder Trägerstoff umfasst.

## Revendications

1. Composé qui est : (i) 5-cholesten-3, 25-diol, disulfate (25HCDS) de formule 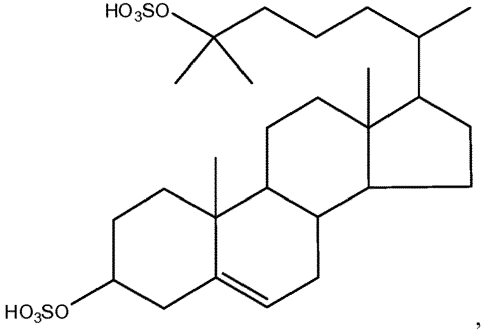 ou (ii) sel pharmaceutiquement acceptable de celui-ci ; destiné à une utilisation en tant que médicament.

2. Composé destiné à une utilisation selon la revendication 1, le composé étant

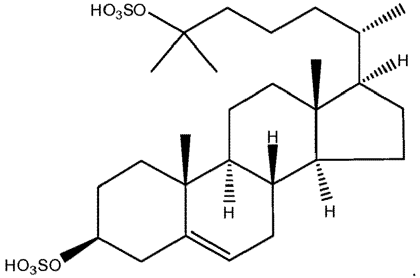

3. Composé selon la revendication 1 ou 2 destiné à une utilisation dans un procédé de : réduction de lipides chez un sujet en ayant besoin ; réduction de la biosynthèse du cholestérol et des lipides chez un sujet en ayant besoin ; réduction d'une inflammation chez un sujet en ayant besoin ; traitement du diabète chez un sujet en ayant besoin ; traitement de l'hyperlipidémie chez un sujet en ayant besoin ; le traitement de l'athérosclérose chez un sujet en ayant besoin ; traitement de la stéatose hépatique chez un sujet en ayant besoin ; ou traitement d'une maladie inflammatoire chez un sujet en ayant besoin.

4. Composé en vue d'une utilisation selon la revendication 3, dans lequel le procédé comprend :
l'administration du composé dans une quantité allant de 0,1 mg/kg à 100 mg/kg en fonction de la masse corporelle du sujet, ou l'administration du composé dans une quantité allant de 1 mg/kg à 10 mg/kg, en fonction de la masse corporelle du sujet ; et/ou
l'administration du composé par voie orale, par voie entérale, par voie sublinguale, par voie transdermique, par voie intraveineuse, par voie péritonéale, par voie parentérale, par injection, par injection sous-cutanée et/ou par injection intramusculaire.

5. Composé selon la revendication 1 ou 2.

6. Composé selon la revendication 5, qui est un composé isolé.

7. Composé selon la revendication 5 ou 6, qui est pratiquement pur.

8. Composé selon l'une quelconque des revendications 5 à 7, qui est sous forme solide.

9. Composé selon la revendication 8, qui est :
sous forme de poudre ; et/ou
sous forme lyophilisée.

10. Composition pharmaceutique comprenant : (i) un composé tel que décrit dans la revendication 1 ou 2 ; et (ii) un excipient, diluant ou support physiologiquement acceptable.

11. Composition pharmaceutique selon la revendication 10, la composition étant formulée sous forme de dosage unitaire.

12. Composition pharmaceutique selon la revendication 10 ou 11, dans laquelle :
la composition est sous forme solide ; ou
la composition est sous forme solide et la composition est sous forme de poudre, de comprimé, de gélule ou de pastille ; ou
la composition est sous forme solide et la composition comprend le composé sous forme lyophilisée conjointement avec un agent diluant, la composition étant éventuellement dans un flacon hermétique, une ampoule, une seringue ou un sac.

13. Composition pharmaceutique selon la revendication 10 ou 11, qui comprend un support qui est un liquide et éventuellement dans laquelle :
le composé est solubilisé dans ledit liquide ou dispersé dans ledit liquide ; et/ou
ledit liquide est aqueux ; et/ou
ledit liquide est l'eau stérile destinée à des injections ou une solution saline tamponnée au phosphate ; et/ou
ladite composition est dans un flacon hermétique, une ampoule, une seringue ou un sac.

14. Procédé de production d'un composé selon la revendication 1 ou 2, lequel procédé comprenant la réaction de 25-hydroxycholestérol avec une source de trioxyde de soufre et, éventuellement, la formation d'un sel pharmaceutiquement acceptable à partir de 5-cholesten-3, 25-diol, disulfate (25HCDS) obtenus, la source de trioxyde de soufre étant éventuellement un complexe d'amine de trioxyde de soufre.

15. Procédé de production d'une composition pharmaceutique selon l'une quelconque des revendications 10 à 13, lequel procédé comprenant la combinaison dudit composé avec ledit excipient, diluant ou support physiologiquement acceptable.
